# EUROPEAN PATENT SPECIFICATION

(11) **EP 3 008 470 B1**
(45) Date of publication and mention of the grant of the patent: **21.03.2018**
(21) Application number: 14738621.3
(22) Date of filing: 12.06.2014
(51) Int. Cl.: G01N 33/68

(54) **METHOD FOR DIAGNOSING AN INCREASE OF LOCAL AND/OR SYSTEMIC BONE RESORPTION**
VERFAHREN ZUR DIAGNOSE DER ERHÖHUNG DER LOKALEN UND/ODER SYSTEMISCHEN KNOCHENRESORPTION
PROCÉDÉ DE DIAGNOSTIC D'UNE AUGMENTATION D'UNE RÉSORPTION OSSEUSE LOCALE ET/OU SYSTÉMIQUE

(30) Priority: 13.06.2013 IT MI20130974
(43) Date of publication of application: 20.04.2016
(73) Proprietor: Istituto Ortopedico Rizzoli, 40136 Bologna (IT)
(72) Inventor: FINI, Milena, 40068 San Lazzaro Di Savena (Bologna) (IT); GIARDINO, Roberto, 40137 Bologna (IT); SALAMANNA, Francesca, 40127 Bologna (IT)
(74) Representative: Errico, Michela
(86) International application number: PCT/IB2014/062164
(87) International publication number: WO 2014/199331

(56) References cited:
- DEL FATTORE ET AL: "Genetics, pathogenesis and complications of osteopetrosis", BONE, PERGAMON PRESS., OXFORD, GB, vol. 42, no. 1, 20 December 2007 (2007-12-20), pages 19-29, XP022397309, ISSN: 8756-3282
- SALAMANNA FRANCESCA ET AL: "MRMT-1 rat breast carcinoma cells and models of bone metastases: improvement of an in vitro system to mimic the in vivo condition.", ACTA HISTOCHEMICA JAN 2013, vol. 115, no. 1, January 2013 (2013-01), pages 76-85, XP002713112, ISSN: 1618-0372
- MCCORMICK R KEITH: "Osteoporosis: integrating biomarkers and other diagnostic correlates into the management of bone fragility.", ALTERNATIVE MEDICINE REVIEW : A JOURNAL OF CLINICAL THERAPEUTIC JUN 2007, vol. 12, no. 2, June 2007 (2007-06), pages 113-145, XP002713113, ISSN: 1089-5159
- Kini U and Nandeesh BN: "Physiology of Bone Formation, Remodeling, and Metabolism" In: Fogelman I, Gnanasegaran G, Wall H: "Radionuclide and Hybrid Bone Imaging", 2012, Springer, XP002713114, pages 29-57, the whole document

## Description

The present invention relates to a method for diagnosing of a local and/or systemic increase in bone resorption or a pathological condition associated therewith, preferably selected in the group consisting in: osteoporosis, osteopenia and osteolysis.

Moreover, the method of the present invention makes it possible to prevent and/or limit the complications associated with said pathological condition, for example fractures, preferably pathological, deformities, chronic pain, impaired balance or impaired quality of life.

Bone resorption is part of the biological bone remodeling activity mainly performed by osteoblasts and osteoclasts.

Osteoclasts are large multinucleated cells belonging to the monocyte-macrophage lineage, deriving, that is, from hematopoietic mesenchymal cells. Osteoclasts have the function of resorbing bone by eroding it through enzymes of exocytosis in an acidic environment, collaborating with calcium homeostasis.

Osteoblasts are the cells that participate directly in the formation of bone tissue by secreting the organic components of the matrix (for example collagen, proteoglycans and glycoproteins) and regulating the deposition of mineral salts.

An increase in osteoclastic activity and/or a decrease in osteoblastic activity in an individual causes a reduction in bone mass and, consequently, a increase in fragility and in the risk of fractures.

Examples of pathological conditions correlated with an increase in bone resorption are osteoporosis, osteopenia or osteolysis.

Of particular relevance is osteoporosis, which identifies a degenerative disease of the skeleton, characterized by a reduced bone mass caused by a variety of factors, for example nutritional, metabolic, hormonal and genetic ones or factors secondary to other pathologies or else improper lifestyles. In these conditions, due to the reduction in density and alterations in bone microarchitecture, the skeleton is subject to a greater risk of pathological or fragility fractures. Consequently, this type of pathology, if not adequately treated, determines a decrease in the quality of life of the affected individual, who suffers, in particular, from the complications due to fractures and microfractures; these affect, in particular, the rachis, the proximal femur and wrist.

Those particularly exposed to osteoporosis are patients of an advanced age and women after menopause in whom an imbalance between bone resorption and formation manifests itself. In women, this process is mainly caused by the deficiency of estrogens, which in themselves have a protective action on bone tissue. The total amount of bone mass a woman has upon reaching menopause is essential for protecting against postmenopausal bone loss.

According to data of the World Health Organization (WHO), osteoporosis affects over 75 million people in the United States, Europe and Japan. In the United States and in the European Union, approximately 30% of postmenopausal women are affected by osteoporosis and it is estimated that, among these, over 40% will sustain a fracture during their lifetime. In Italy, the prevalence of osteoporosis in women over seventy years of age is approximately 50%, with about 4 million women affected by the pathology. The data regarding femoral fractures are even more worrying: the number of hospital admissions is estimated to be about 70,000 per year, and the incidence of this phenomenon is rising.

According to further studies, 50% of women and 20% of men have an osteoporotic fracture after the age of 50 and 2,000,000 fractures are sustained each year, with 350,000 fractures of the femoral neck and a mortality of 20%, resulting in costs of about 19 billion dollars a year.

These epidemiological data are worrying as regards both people's health and the impact of the costs of these pathological conditions on the public health care system. Thus there is a greatly felt need to be able to make an early determination, in a fast, reliable and cost-effective manner of the pathological conditions associated with bone resorption, such as osteoporosis, osteopenia and osteolysis.

For many years, osteoporosis was determined on the basis of clinical detection of a pathological fracture and radiological evidence of a severe decline in bone density, whether accompanied or not by fractures.

However, over time a need has been perceived to switch from qualitative methods of investigation to quantitative methods. Accordingly, techniques have been developed to measure bone density, e.g. single- and dual-photon beam computerized bone absorptiometry (CBA), digital dual-energy X-ray absorptiometry (DEXA), quantitative computed tomography (QCT) and bone ultrasonography.

According to the World Health Organization (WHO), DEXA represents the gold standard for identifying conditions associated with bone resorption, such as osteoporosis and osteopenia.

DEXA is a method that provides values related to various body regions and is based on the principle of differential attenuation of an X-ray beam on two energy levels as it passes through tissues. The radiation a patient is exposed to during DEXA ranges from around 5 to 10 mRem depending on the facility. The reported costs amount to around 50 dollars in the US and the average direct costs to the health care system in Italy are reported to be 30 euros.

However, DEXA is a technique that has several limits where the aim is to undertake a complete, periodic screening of the whole population, both because of the type of equipment used (not transportable), and because performing the test requires dedicated rooms. In fact, it is necessary to highlight that the world population is increasingly "an aging population", characterized, that is, by a higher and higher average age, so regular mass screening is fundamental. Moreover, numerous pathologies, improper lifestyles and increasingly widespread pharmacological treatments (for example, chronic intake of cortisone-based drugs and anticoagulants), contribute to significantly increasing the prevalence and incidence of conditions associated with bone resorption.

Moreover, it takes around 15-30 minutes to perform DEXA (the time varies proportionately with the size of the subject and the facility concerned) and the test provides a two-dimensional density measurement calculated over an area, not over a volume.

In particular, DEXA on the spinal column and hip is sensitive to degenerative changes and individuals with substantial degenerative pathologies could show a high area density which suggests a lower risk of fractures than is actually present. Moreover, all the structures near the spinal column can influence the measurements of BMD.

Finally, DEXA on its own is not a test capable of exactly predicting the risk of fractures.

In pathological conditions associated with an increase in bone resorption, biochemical and biohumoral parameters are also evaluated. The aim is both to determine the pathology and to define the most appropriate pharmacological treatment and response to a drug or to differentiate a pathological condition with a high bone turnover from one with a low bone turnover. In fact, the basic tests that are generally performed include, for example: calcemia, phosphatemia, level of intact parathyroid hormone (PTH), cortisol, free testosterone, somatotropin, 25-OH-vitamin D3, 24 hour calciuria and phosphaturia, bone alkaline phosphatase and electrophoretic protidogram. Whereas the more specific tests that are habitually performed include, for example: resorption markers such as acidic phosphatase (serum), hydroxyproline (urine, serum), pyridinoline (urine, serum), urinary deoxypyridinoline (DPD) (urine), N-terminal telopeptide of type I collagen (NTX-I) (urine, serum), C-terminal of telopeptide of type I collagen (CTX-I) (urine, serum); formation markers such as alkaline phosphatase (ALP) (serum), bone alkaline phosphatase (bALP)(serum), osteocalcin (BGP) (serum), C-terminal propeptide of type I procollagen (serum, urine) and N-terminal propeptide of type I procollagen (serum, urine).

DEL FATTORE ET AL, 2007 describes histopathological features in an osteopetrosis biopsy. The osteoclasts do not show major morphological defects but they seem unable to form the ruffled border which is indispensable for the resorbing activity. This document however does not verify the differentiation of the monocyte macrophage cell population into osteoclastic cells.

SALAMANNA FRANCESCA ET AL, 2013 reveals the isolation and culturing of peripheral blood mononuclear cells of Sprague Dawley rats and the observation of their differentiation into osteoclasts either due to cocultivation with breast cancer cells or due to treatment with conditioned medium. However, this document does not verify the differentiation of the monocyte macrophage cell population into osteoclastic cells and belongs to a different technical field, namely to a breast cancer metastasis model.

MCCORMICK R KEITH, 2007 is a review that suggests a PRATIA strategy involving 1) a specific diet to reduce the risk of fractures (very frequent in osteoporosis) and 2) to use biomarkers to monitor the metabolic changes.

Kini and Nandeesh, 2012 is a review on the physiology, remodeling and metabolism of bone development.

Based on what has been described above, there is still a greatly felt need to develop reliable, noninvasive, low-cost methods capable of determining, in a timely manner, a pathological condition associated with an increase in bone resorption, such as osteoporosis, osteopenia or osteolysis and thus also a method for preventing the complications associated with these conditions, such as fractures.

The above-mentioned needs of the prior art are met by the method of the present invention, which makes it possible to determine an increase in bone resorption and/or a pathological condition associated therewith, such as osteoporosis, osteopenia or osteolysis in a reliable and timely manner and at a low cost. Moreover, the method of the invention makes it possible to prevent and/or limit the complications associated with these pathological conditions, for example fractures, deformities, chronic pain or impaired balance.

Compared to the prior art methods, the method of the present invention enables a simple, fast diagnosis. In fact, the method of the present invention is carried out on a biological sample isolated from an individual suspected to be at risk for or affected by a pathological condition associated with an increase in bone resorption and envisages verifying whether the monocyte-macrophage cells obtained from the sample spontaneously differentiate into osteoclasts.

In particular, the Applicant has surprisingly found that monocyte-macrophage cells isolated from an osteoporotic biological sample (in particular a blood sample) and cultured in vitro spontaneously differentiate into osteoclasts. In other words, the monocytes-macrophages isolated from biological samples of individuals affected by a pathology associated with a condition of increased bone resorption, when cultured in vitro, differentiate into osteoclasts without there being any need to supplement the culture medium with factors capable of inducing osteoclastic differentiation.

The method of the present invention also has a preventive character, in the sense that, based on the results, further investigations may or may not be undertaken in order to determine a local or systemic alteration in bone metabolism.

In fact, only in the event that the method of the present invention gives a positive result, further instrumental analyses such as DEXA, capable of identifying the specific region affected by an alteration in bone remodeling, can subsequently be carried out.

The fact that, in order to determine a widespread condition of bone resorption, such as, for example, osteoporosis, more costly instrumental analyses like DEXA can be subordinated to the method of the invention, which is simple, fast and economical, constitutes an ideal condition for broad screening of the population. In particular, this enables the use of the instrumental analyses to be restricted to subjects testing positive to the method of the invention, resulting in considerable savings in public health care costs and enhanced precision in predicting the risk of complications from the altered local or systemic bone remodeling.

The present invention will be described in a detailed manner below, also with the aid of the following figures, in which:

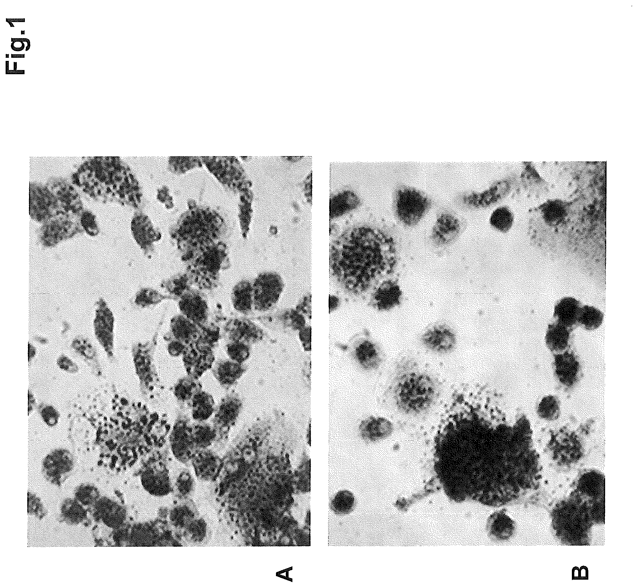

- Figure 1 shows an image acquired with an optical microscope following TRAP staining of osteoclasts spontaneously differentiated starting from monocyte-macrophage cells isolated from osteoporotic rats and cultured in a culture medium devoid of factors capable of inducing osteoclastic differentiation;

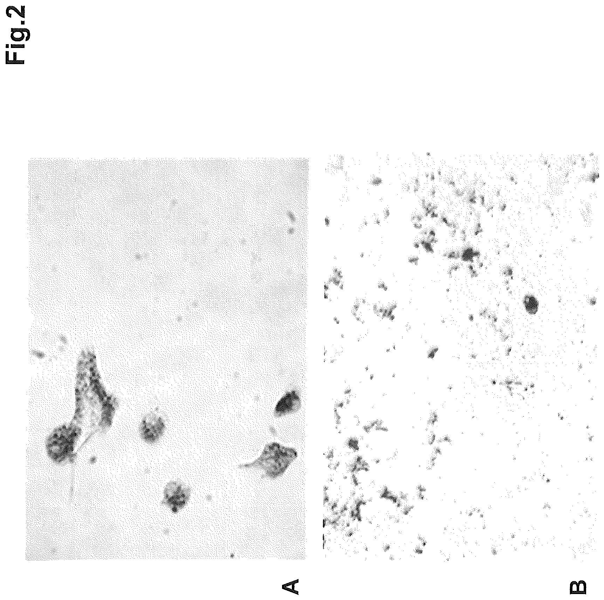

Figure 2 shows an image acquired with an optical microscope following TRAP staining of osteoclasts spontaneously differentiated starting from monocyte-macrophage cells isolated from healthy rats and cultured in a culture medium devoid of factors capable of inducing osteoclastic differentiation.A first aspect of the present invention refers to an in vitro method for determining, in an individual, a pathological condition selected from: osteoporosis, osteopenia, osteolysis, osteolytic bone metastases and bone resorption around implants, said method comprising the steps of:
   (i) Isolating a monocyte-macrophage cell population from a sample obtained from blood, hemoderivative or tissue isolated from an individual suspected to have at least one body region affected by said pathological condition
   (ii) Seeding said monocyte-macrophage cell population in a container comprising a medium suitable for culturing cells; and
   (iii) Verifying the differentiation of said monocyte-macrophage cell population into osteoclastic cells in said container after a predetermined time;
   Wherein said differentiation is indicative of the fact that said individual is affected by said pathological condition.

Preferably, step (i) is performed by disaggregating the biological sample, preferably by using mechanical means and/or by using enzymes; or isolating the elements of the biological sample based on density gradient, or by flow cytofluorimetry.

More preferably, the biological sample is diluted, preferably 1:1, with a buffer before said step (i).

According to a preferred embodiment of the invention, the monocyte-macrophage cell population is seeded at a density ranging from 10² to 10⁷, preferably from 10⁴ to 10⁵ cells/cm².

According to a further preferred embodiment of the invention, the culture medium comprises a quantity of an animal serum ranging from 5 to 20%. According to a further preferred embodiment of the invention, the predetermined time is about 3 days, and/or 7 days and/or about 14 days and/or about 21 days.

According to a further preferred embodiment of the invention, the step (iii) is performed optically or by using at least one antibody directed against at least one osteoclast-specific marker, preferably selected from TRAP and/or cathepsin K; or by colorimetric assay, preferably using phalloidin and/or DAPI.

According to a further preferred embodiment of the invention, the method is applied in combination with at least one traditional biochemical test or with at least bone density measurement method.

The method of the present invention makes it possible to determine an increase in bone resorption activity in an individual, preferably on a local and/or systemic level. In particular, the method of the invention makes it possible to determine, in an individual, a pathological condition associated with an increase in bone resorption activity. Preferably, said pathological condition is selected from among: osteoporosis, osteopenia and osteolysis, in particular peri-implant and/or metastatic osteolysis.

Alternatively and/or in addition, the method of the present invention may be defined as a preventive method in the sense that it enables an evaluation of an individual's risk of being affected by an increase in bone resorption activity or of a pathological condition associated therewith. Moreover, the method of the present invention also makes it possible to prevent and/or of limit the complications associated with said pathological conditions, for example fractures, deformities, chronic pain, aseptic implant loosening, impaired balance or impaired quality of life.

The method of the present invention is a method carried out in vitro and comprises or consists essentially in the steps of:
(i) isolating a monocyte-macrophage cell population from a biological sample obtained from blood, hemoderivative or tissue isolated from an individual suspected to have at least one body region affected by said pathological condition
(ii) seeding said monocyte-macrophage cell population in a container comprising a medium suitable for culturing cells; and
(iii) verifying the presence of osteoclastic cells in said container after a predetermined time.

In the context of the present invention, an increase in bone remodeling activity means an increase in skeletal turnover, i.e., an increase in the activity of breaking down bone relative to the formation of new bone. Normally, the most affected part is the trabecular component of bone, also with a reduction in cortical thickness and alterations in the internal microarchitectural structure. This leads to a deterioration in the mechanical properties of the bone structure and simultaneously reduces its resistance. Therefore, an increase in bone remodeling activity also means an alteration in bone metabolism.

In particular, the increase in bone resorption activity can be systemic or local.

In the context of the present invention, pathological condition associated with an increase in bone remodeling activity means an increase in skeletal turnover. Preferably, said condition associated with bone remodeling is selected from among: osteoporosis, osteopenia, osteolysis, osteolytic bone metastases and bone resorption around implants, for example osteolysis and aseptic loosening.

In the context of the present invention, monocyte-macrophage cell population means a cell population that is generally present in the blood and/or hemoderivatives and is part of the leukocytes (so-called white blood cells). They are present in peripheral blood in a percentage that may vary from 2% to 8% relative to the total number of leukocytes. Monocytes commonly have a diameter of from 9 to 12 µm and anatomically appear with a nucleus that is eccentric in position and can be oval or even kidney shaped, whereas in subjects of a more advanced age it can take on a typical horseshoe shape.

The biological sample according to step (i) is isolated from any region of a human and/or animal body. Preferably, said biological sample is venous, arterial or bone marrow blood, or else a hemoderivative, for example plasma or serum. Preferably, the blood is venous and peripheral, or arterial, more preferably the blood is peripheral and/or bone marrow blood. The separation of the monocyte-macrophage cells according to step (i) is achieved using any technique for separating cell populations known to the person skilled in the art. For example, the cells can be isolated by flow cytofluorimetry (cell sorter).

In particular, with regard to non-liquid biological samples, for example a tissue sample, the monocyte-macrophage cells can be isolated from the sample by disaggregation of the sample itself and subsequent seeding in containers containing a culture medium, preferably comprising an animal serum.

The disaggregation of the sample can be of a mechanical type, for example the starting sample can be finely minced with a scalpel or scissors in a culture medium conducive to the growth of monocyte-macrophage cells. Subsequently, the minced sample is made to pass through sieves, preferably with progressively finer meshes, or with needles of decreasing size or simply through pipettes in such a way as to eliminate the aggregates of larger dimensions.

Alternatively, the disaggregation can be enzymatic, i.e., the starting sample is treated by means of a solution containing digestive enzymes, for example trypsin and/or collagenase, i.e., enzymes that act at the level of the junctions between cells and/or between cells and the extracellular matrix.

In a preferred embodiment of the invention, when the biological sample is a blood or hemoderivative sample, the step of isolating the monocyte-macrophage cell population is preferably carried out by separating the various elements of the biological sample based on density, more preferably by density gradient separation. In this case, the various constituent elements of the sample can be separated by sedimentation, preferably using a centrifuge. A density gradient separation of a polymer, such as Histopaque or Ficoll, is particularly useful for these purposes.

In any case, the skilled person can use any method known in the art for the purpose of isolating the monocyte-macrophage cell population from the isolated blood or hemoderivative sample.

Preferably, before undergoing the separation step, the biological sample, be it blood or a hemoderivative, is diluted with a buffer solution, more preferably in a 1:1 ratio (that is, one volume of sample per volume of buffer).

The isolated monocyte-macrophage cell population is subsequently seeded in a container in the presence of a medium suitable for culturing cells, that is, a culture medium. Synonyms of culture medium are growth medium or expansion medium, that is, a solution enabling monocyte-macrophage cells to proliferate, i.e., to expand numerically.

In the context of the present invention, the term seeding means the procedure routinely used in the field of the cell cultures to maintain or amplify cells in vitro. In particular, this procedure envisages that the cells, after being counted, are usually re-suspended in a liquid culture medium and distributed inside a flat-bottomed plastic or glass container at a given density (i.e., a given number of cells per milliliter or per cm², depending on whether the cells are growing in suspension or growing in adhesion).

The density at which the cells are seeded and the culture medium they are seeded in are specific for each type of cell.

Preferably, in the method of the present invention the cells are seeded at a density of 10²-10⁷ cells per cm², preferably 10⁴-10⁵ cells per cm².

The container can be any container generally used for cell cultures, for example Petri dishes, plates or flasks, of various sizes and materials, depending on how many cells are available and/or how many cells it is desired to seed or obtain.

The culture medium serves to nourish the cells during their growth (proliferation) and in fact it contains a quantity, which varies with the cell type, of supplementary factors, for example salts, essential and non-essential amino acids, buffers, serum, growth factors etc..

Preferably, in the present invention the culture medium comprises a liquid medium (so-called basal medium), for example, Dulbecco's Modified Eagle Medium (DMEM), DME-F12, RPMI-1640, alpha-modified Eagle Medium, HAM'S Nutrient Mixture, Medium 199. The basal medium is preferably supplemented with an animal serum, for example fetal calf serum. The serum concentration varies preferably from 5 to 20%, more preferably from 8 to 12%, even more preferably it is about 10%. For example, in the latter case it means that 10 milliliters of serum is added to the basal liquid medium to obtain a final volume of 100 milliliters of culture medium.

Moreover, the culture medium can further contain a mixture of antibiotics, preferably the antibiotics generally used for cell cultures, in particular a solution of penicillin and/or streptomycin. The concentration of antibiotics in the culture medium varies from 80 to 120 Units (U)/ml, preferably about 100 U/ml of penicillin and/or an equal quantity of streptomycin. Moreover, the culture medium can further contain a buffer solution, which, preferably, is HEPES (i.e., 4-(2-hydroxyethyl)-1-piperazineethanesulfonic acid). Preferably, the buffer is HEPES at a concentration that varies from 8 to 12 g/L; preferably, it is about 9.94 g/L.

In a particularly preferred embodiment, the culture medium comprises a medium supplemented with animal serum, the antibiotic and, possibly, also a buffer as described above. More preferably, the culture medium is devoid of agents capable of inducing the differentiation of a cell into an osteoclast. In other words, the culture medium is not supplemented with the factors necessary for differentiation toward the osteoclastic phenotype used in the field of cell cultures. Examples of such factors are: RANKL, (Receptor Activator of Nuclear Factor Kappa-B Ligand), M-CSF (Macrophage Colony-Stimulating Factor) or PTH (Parathyroid Hormone). According to a preferred aspect of the invention, the culture medium is replaced with fresh culture medium after 1 and/or 3 and/or 7 and/or 14 and/or 21 days after the adhesion of cells to the container.

After cell adhesion, the monocyte-macrophage cells undergo the step of verifying the spontaneous differentiation into osteoclasts. In particular, the verification step is carried out about 3 days and/or 7 days and/or 14 days, and/or 21 days after adhesion.

The verification step, i.e. step of verifying the differentiation of the monocyte-macrophage cells into osteoclasts, can be carried out visually, for example with the aid of an optical and/or electronic microscope. In fact, by observing the morphology of the cells under a microscope, the skilled person will be able to say whether the monocyte-macrophage cells have differentiated into osteoclasts. In fact, osteoclasts derive from the fusion of monocytes and thus appear as multinucleated cells of larger dimensions than monocytes.

The differentiation of monocyte-macrophage cells may occur only in the case of a biological sample isolated from an individual having at least one body region affected by an increase in bone resorption, or by an individual affected by a pathological condition associated with an increase in bone resorption, such as osteoporosis, osteopenia or osteolysis. In fact, if the method of the invention is applied on a biological sample isolated from a healthy individual, i.e., an individual who has no body region affected by an increase in bone resorption or pathologies associated therewith, the monocyte-macrophage cells isolated from the biological sample and cultured as described above will not differentiate into osteoclasts but will rather be destined to die, like all terminally differentiated cells. Therefore, in this case, over time one would not find cells adhered to the inside of the container, since following their death, the monocyte-macrophage cells would come detached from the container and be carried away during the washing cycles.

Alternatively, or in addition, the differentiation of the monocyte-macrophage cells into osteoclasts can be verified/detected: using antibodies, preferably conjugated and/or marked, directed against specific markers of osteoclasts, preferably selected from among: tartrate-resistant acid phosphatase (TRAP) and/or cathepsin K; or by colorimetric assay, preferably using phalloidin, possibly associated with DAPI (4',6-diamidino-2-phenylindole).

Phalloidin associated with DAPI represents a colorimetric assay enabling a specific structure to be revealed by emission of a fluorescent signal. Phalloidin binds to polymerized actin, and reveals the actin microfilaments, whilst DAPI specifically binds to DNA. After staining with phalloidin, the osteoclasts exhibit a peculiar ring-like arrangement of the actin filaments. By binding to DNA, DAPI moreover reveals the multinucleated nature of the osteoclasts.

Alternatively, or in addition, the differentiation of the monocyte-macrophage cells into osteoclasts can be verified/detected by measuring the levels of expression and/or activation of the specific proteins of bone metabolism, preferably tartrate-resistant acid phosphatase (TRAP) or cathepsin K. For example, the expression levels can be evaluated by means of PCR, RT-PCR, real-time PCR, microarray or any other method know to the person skilled in the art which serves that purpose.

According to a further aspect of the invention, the method can be carried out in combination with at least one biochemical test of a traditional type and/or with at least one traditional method for measuring bone density used to determine an increase in bone remodeling, and in particular for diagnosing a pathology associated therewith, preferably selected from among: osteoporosis, osteopenia, osteolytic bone metastases and bone resorption around implants, for example osteolysis and aseptic loosening. Examples of a biochemical test of a traditional type are: calcaemia, phosphatemia, level of intact parathyroid hormone (PTH), cortisol, free testosterone, somatotropin, 25-OH-vitamin D3, 24 hour calciuria and phosphaturia, bone alkaline phosphatase, electrophoretic protidogram, resorption markers such as acidic phosphatase (serum), hydroxyproline (urine, serum), pyridinoline (urine, serum), urinary deoxypyridinoline (DPD) (urine), N-terminal telopeptide of type I collagen (NTX-I) (urine, serum), C-terminal of telopeptide of type I collagen (CTX-I) (urine, serum); formation markers such as alkaline phosphatase (ALP) (serum), bone alkaline phosphatase (bALP)(serum), osteocalcin (BGP) (serum), C-terminal propeptide of type I procollagen (serum, urine) or N-terminal propeptide of type I procollagen (serum, urine).

Examples of a traditional method for measuring bone density are: single- and dual-photon beam computerized bone absorptiometry (CBA), digital dual-energy X-ray absorptiometry (DEXA), quantitative computed tomography (QCT) and bone ultrasonography.

A further aspect of the present invention relates to a kit for implementing the method of the invention, said kit comprising reagents and consumables for carrying out the various steps of the method, preferably: sterilized vials preferably comprising at least one anticoagulant, preferably EDTA; a solution for isolating the monocyte-macrophage cells from the biological sample via density gradient separation, at least one basal medium, and/or at least one serum, and/or at least one antibiotic for culturing monocyte-macrophage cells; and, optionally, plates for culturing monocyte-macrophage cells, preferably plates with 6-98 wells.

In addition, the kit can comprise cell wash solutions, for example PBS or analogous solutions and a vital stain for cell counts, preferably trypan blue. In a further embodiment of the present invention, the kit can comprise at least one antibody directed against at least one osteoclast marker, preferably an antibody, possibly conjugated and/or marked, directed against TRAP and/or cathepsin K.

In a further embodiment of the present invention the kit can comprise at least one stain, preferably selected from among DAPI and/or phalloidin.

In a preferred embodiment, the kit comprises a ready-to-use cell culture medium. Said culture medium preferably comprises a basal medium, at least one animal serum and, optionally, at least one antibiotic. The culture medium is preferably lyophilized and ready to be reconstituted at the time of use.

In a further embodiment, the kit comprises vials with a pre-measured quantity of vital stain, preferably lyophilized.

### EXAMPLE

The method according to the invention was applied on blood samples obtained from healthy female rats and female rats in which osteoporosis was induced by bilateral oophorectomy.

In particular, 6 healthy female rats and 6 osteoporotic rats were tested. Each blood sample corresponds approximately to at least 2cc of blood.

In this case a 2cc blood sample was taken.

The blood sample is placed inside a sterile vial containing ethylenediaminetetraacetic acid (EDTA).

Within twenty-four hours after the blood sample is taken, it is diluted 1:1 under sterile conditions inside the vial containing EDTA 1:1 with a 10X phosphate buffered saline solution (PBS) at a concentration of 100 ml/L at pH 7.4.

It is advisable to carry out all the steps under conditions of sterility, for example by performing the treatment under a laminar flow hood. Thereafter, a volume of Histopaque 1077 or Ficoll separation medium or, alternatively, an aqueous solution of high molecular weight sodium diatrizoate and polysaccharide is prepared under sterile conditions in a sterile vial, in which two volumes of the blood sample diluted as described above are very slowly stratified. The blood thus treated is centrifuged in a refrigerated benchtop centrifuge for thirty minutes at room temperature at 400g, with the instrument acceleration and deceleration set to a value of one.

In this manner, the blood cells become stratified based on density. In particular, erythrocytes, having a greater density, pass through the separation layer and settle on the bottom; the polymorphonucleated cells become stratified, forming a fine whitish layer above the layer of red blood cells; the mononucleated cells of peripheral blood, being less dense than the polymer, collect in the interface between the plasma and the separation polymer, forming a whitish ring.

The upper layer of the sample is removed with a sterile pipette and discarded until one arrives at about 0.5 centimeters from the opaque interface containing the mononucleated cells.

At this point the ring of cells containing the mononucleated cells is transferred with a sterile pipette into a new sterile vial, to which about ten milliliters of PBS are added. The sample is then centrifuged at 250g for ten minutes at room temperature. At the end of centrifugation the supernatant is aspirated and discarded.

The pellet thus obtained is resuspended in five milliliters of PBS, mixed and centrifuged at 250 g for ten minutes at room temperature.

At the end of centrifugation, the supernatant is aspirated and discarded. At this point the pellet obtained is again resuspended in five milliliters of PBS and again centrifuged at 250g for ten minutes at room temperature.

At the end of this last centrifugation, the pellet is resuspended in one milliliter of a solution consisting of: 10% fetal calf serum (FCS), 1% of a penicillin - streptomycin solution and 25mM of HEPES, the volume being brought to 100% with Dulbecco's Modified Eagle Medium.

An aliquot (10µl) of the cell suspension (pellet resuspended in DMEM) is placed in a new sterile vial with 50µl of Trypan Blue solution and 40µl of physiological solution. An aliquot (10µl) of the solution thus obtained is withdrawn and transferred into the hemocytometer chamber in order to count the cells present.

The number of live (transparent) cells and the number of dead cells (blue due to the absorption of the Trypan Blue) is determined under an optical microscope at 10x magnification with the aid of a Neubauer chamber, a chamber made up of 9 medium squares including a number of counting squares, arranged in a cross, equivalent to 5. Each of them holds a volume of 0.1 µl, so that it is sufficient to multiply by 10,000 to compute the cells per ml.

Once the cells are counted, the remaining cell suspension is seeded in 24-well plates at a concentration of 3 x10⁶ cells/cm² in the presence of DMEM medium supplemented with 10% FCS, 1% penicillin - streptomycin solution and HEPES.

At this point the cells seeded in the plates are placed in an incubator at a temperature of 37°C and in the presence of a CO₂ pressure of 5% in order enable cell adhesion.

The cell culture medium (DMEM+ 10% FCS+1% penicillin - streptomycin solution + 25mM HEPES) is replaced 24 hours after cell adhesion. Differentiation toward the osteoclastic phenotype is evaluated starting from 7 to 14 days after cell adhesion using both qualitative methods and quantitative methods.

After 7-14 days, through observation under an optical microscope, it is possible to establish the presence of alterations in the monocytes which are indicative of osteoporosis, osteopenia or in any case a state of altered systemic or local bone resorption.

At the different experimental times taken into consideration (7, 14 and 21 days) the monocytes-macrophages isolated from the blood sample obtained from osteoporotic rats show a spontaneous differentiation toward the osteoclastic phenotype. In contrast, the monocytes-macrophages obtained from the blood sample of healthy animals (controls) do not differentiate into osteoclasts and die (viability equal to 0%).

The differentiation of the monocytes-macrophages obtained from blood samples of osteoporotic rats into osteoclasts was confirmed with: an enzymatic study of the expression of the marker of osteoclastic activity TRAP/ALP by staining with phalloidin associated with DAPI (4',6-diamidino-2-phenylindole), and by measuring the levels of expression and activation of the specific proteins (of the enzymes) of bone metabolism (tartrate-resistant acid phosphatase - TRAP) or cathepsin K (selective biomarkers of osteoclastic activity);

Therefore, the above-described tests surprisingly reveal that, contrary to what occurs in healthy rats (i.e. ones not in pathological conditions), the monocytes isolated from the blood of osteoporotic rats spontaneously differentiate into osteoclasts, even in the absence of any factor capable of stimulating cellular differentiation toward the osteoclastic phenotype. Moreover, the monocytes-macrophages isolated from blood of osteoporotic rats show a significantly greater cell viability (measured at 7, 14 and 21 days after seeding) than those isolated from peripheral blood of healthy rats. In fact, 7 days after seeding, the monocytes-macrophages isolated from healthy rats show a cell viability of 0%, in accordance with what has been reported in the literature. In fact, some studies have demonstrated that the monocytes-macrophages isolated from healthy subjects and cultured without factors capable of inducing osteoclastic differentiation (such as the Receptor Activator of Nuclear factor Kappa-B Ligand (RANKL), Macrophage Colony-Stimulating Factor M-CSF or Parathyroid Hormone (PTH)) are not able to differentiate spontaneously into osteoclasts and lose vitality.

The osteoclastic differentiation of the monocytes-macrophages obtained from blood samples of osteoporotic rats was confirmed by the positive qualitative and quantitative result of the tartrate-resistant acid phosphatase (TRAP) test. This experiment revealed osteoclastic differentiation only and exclusively for the monocytes-macrophages obtained from blood samples of osteoporotic rats (Figure 1A and B) at all experimental times.

Finally, a further confirmation of the differentiation of monocytes-macrophages from osteoporotic animals into osteoclasts was provided by subsequent laboratory investigations using scanning electron microscopy (SEM) and fluorescent phalloidin staining associated with DAPI (4',6-diamidino-2-phenylindole), which revealed the presence of well-differentiated osteoclasts originating exclusively from the monocytes-macrophages obtained from blood samples of osteoporotic rats. Therefore, the method for diagnosing osteoporosis is a reliable, very simple and low-cost method. It requires extremely reduced quantities of blood, for example small quantities obtained from routine blood samples taken for yearly screening of the population.

The method of the invention can also be considered as a complement to traditional biochemical tests and could enable the pathology to be diagnosable even earlier than with traditional methods, such as, for example, DEXA, reveal the signs thereof and predict the risk of fractures to which an individual is exposed.

## Claims

1. In vitro method for determining, in an individual, a pathological condition selected from: osteoporosis, osteopenia, osteolysis, osteolytic bone metastases and bone resorption around implants, said method comprising the steps of:
(i) Isolating a monocyte-macrophage cell population from a sample obtained from blood, hemoderivative or tissue isolated from an individual suspected to have at least one body region affected by said pathological condition
(ii) Seeding said monocyte-macrophage cell population in a container comprising a medium suitable for culturing cells; and
(iii) Verifying the differentiation of said monocyte-macrophage cell population into osteoclastic cells in said container after a predetermined time;
Wherein said differentiation is indicative of the fact that said individual is affected by said pathological condition.

2. The method according to claim 1, wherein said step (i) is performed by disaggregating the biological sample, preferably by using mechanical means and/or by using enzymes; or isolating the elements of the biological sample based on density gradient, or by flow cytofluorimetry.

3. The method according to claim 1 or 2, wherein said biological sample is diluted, preferably 1:1, with a buffer before said step (i).

4. The method according to any one of claims 1-3, wherein said monocyte-macrophage cell population is seeded at a density ranging from 10² to 10⁷.

5. The method according to anyone of claims 1-4, wherein the cell density ranges from 10⁴ to 10⁵ cells/cm².

6. The method according to any one of claims 1-5, wherein said culture medium comprises a quantity of an animal serum ranging from 5 to 20%.

7. The method according to any one of claims 1-6, wherein said predetermined time is about 3 days, and/or 7 days and/or about 14 days and/or about 21 days.

8. The method according to any one of claims 1-7, wherein said step (iii) is performed optically or by using at least one antibody directed against at least one osteoclast-specific marker, preferably selected from TRAP and/or cathepsin K; or by colorimetric assay, preferably using phalloidin and/or DAPI.

9. The method according to any one of claims 1-8 in combination with at least one traditional biochemical test or with at least bone density measurement method.

## Patentansprüche

1. In-vitro-Verfahren zur Bestimmung eines pathologischen Zustands in einem Individuum, ausgewählt aus: Osteoporose, Osteopenie, Osteolyse, osteolytischen Knochenmetastasen und Knochenresorption um Implantate, wobei das Verfahren die Schritte umfasst:
(i) Isolieren einer Monozyten-Makrophagen-Zellpopulation von einer Probe, die aus Blut, Hämoderivativ oder Gewebe erhalten wurde, die von einem Individuum isoliert wurde, von dem angenommen wurde, dass es zumindest eine Körperregion aufweist, die vom pathologischen Zustand betroffen ist;
(ii) Säen der Monozyten-Makrophagen-Zellpopulation in einem Behälter, umfassend ein Medium, das zum Kultivieren von Zellen geeignet ist; und
(iii) Verifizieren der Differenzierung der Monozyten-Makrophagen-Zellpopulation in osteoklastischen Zellen im Behälter nach einer vorbestimmten Zeit;
wobei die Differenzierung auf die Tatsache hinweist, dass das Individuum vom pathologischen Zustand betroffen ist.

2. Verfahren nach Anspruch 1, wobei der Schritt (i) durchgeführt wird, indem die biologische Probe disaggregiert wird, vorzugsweise unter Verwendung von mechanischen Mitteln und/oder unter Verwendung von Enzymen; oder durch Isolieren der Elemente der biologischen Probe basierend auf dem Dichtegradienten oder durch Durchflusscytofluorimetrie.

3. Verfahren nach Anspruch 1 oder 2, wobei die biologische Probe vor dem Schritt (i), vorzugsweise 1:1, mit einem Puffer verdünnt wird.

4. Verfahren nach einem der Ansprüche 1 bis 3, wobei die Monozyten-Makrophagen-Zellpopulation in einer Dichte im Bereich von 10² bis 10⁷ gesät wird.

5. Verfahren nach einem der Ansprüche 1 bis 4, wobei die Zelldichte im Bereich von 10⁴ bis 10⁵ Zellen/cm² liegt.

6. Verfahren nach einem der Ansprüche 1 bis 5, wobei das Kulturmedium eine Menge eines Tierserums im Bereich von 5 bis 20% umfasst.

7. Verfahren nach einem der Ansprüche 1 bis 6, wobei die vorbestimmte Zeit etwa 3 Tage und/oder 7 Tage und/oder etwa 14 Tage und/oder etwa 21 Tage beträgt.

8. Verfahren nach einem der Ansprüche 1 bis 7, wobei der Schritt (iii) optisch oder unter Verwendung von zumindest einem gegen zumindest einen osteoklastenspezifischen Marker, vorzugsweise ausgewählt aus TRAP und/oder Cathepsin K, gerichteten Antikörper durchgeführt wird; oder durch kolorimetrische Prüfung, vorzugsweise unter Verwendung von Phalloidin und/oder DAPI.

9. Verfahren nach einem der Ansprüche 1 bis 8 in Kombination mit zumindest einem traditionellen biochemischen Test oder zumindest mit einem Knochendichtemessverfahren.

## Revendications

1. Procédé in vitro servant à déterminer, chez un individu, une condition pathologique sélectionnée à partir de : l'ostéoporose, l'ostéopénie, l'ostéolyse, métastases osseuses ostéolytiques et la résorption osseuse autour d'implants, ledit procédé comprenant les étapes de :
(i) isoler une population cellulaire monocyte / macrophage à partir d'un échantillon sanguin, de dérivés de sang ou de tissu isolé chez un individu suspecté d'avoir au moins une région du corps affectée par ladite condition pathologique ;
(ii) ensemencer ladite population cellulaire monocyte / macrophage dans un récipient comprenant un milieu adapté à la culture de cellules ; et
(iii) vérifier la différentiation de ladite population cellulaire monocyte / macrophage dans les cellules ostéoclastiques dans ledit récipient après une période de temps prédéfinie ;
dans laquelle ladite différentiation est révélatrice du fait que ledit individu est affecté par ladite condition pathologique.

2. Procédé selon la revendication 1, dans lequel ladite étape (i) est réalisée en désagrégeant l'échantillon biologique, de préférence en utilisant des moyens mécaniques et/ou en utilisant des enzymes ; ou en isolant les éléments de l'échantillon biologique en se basant sur le gradient de densité, ou par cytométrie en flux.

3. Procédé selon la revendication 1 ou 2, dans lequel ledit échantillon biologique est dilué, de préférence 1:1, avec une solution tampon avant ladite étape (i).

4. Procédé selon l'une quelconque des revendications de 1 à 3, dans lequel ladite population cellulaire monocyte / macrophage est ensemencée à une densité comprise entre 10² à 10⁷ .

5. Procédé selon l'une quelconque des revendications de 1 à 4, dans lequel la densité de cellules est comprise entre 10⁴ et 10⁵ cellules/cm².

6. Procédé selon l'une quelconque des revendications de 1 à 5, dans lequel ledit milieu de culture comprend une quantité de sérum animal comprise entre 5 et 20 %.

7. Procédé selon l'une quelconque des revendications de 1 à 6, dans lequel ladite période de temps prédéfinie est d'environ 3 jours et/ou 7 jours et/ou environ 14 jours et/ou environ 21 jours.

8. Procédé selon l'une quelconque des revendications de 1 à 7, dans lequel ladite étape (iii) est réalisée de façon optique ou en utilisant au moins un anticorps dirigé contre au moins un marqueur spécifique des ostéoclastes, de préférence sélectionné à partir de la TRAP et/ou de la cathepsine K ; ou un test colorimétrique, de préférence en utilisant la phalloïdine et/ou le DAPI.

9. Procédé selon l'une quelconque des revendications de 1 à 8, en combinaison avec au moins un essai biochimique traditionnel ou avec au moins un procédé de mesure de la densité osseuse.
